**Europäisches Patentamt**

**European Patent Office**

**Office européen des brevets**

(19)

(11) Veröffentlichungsnummer: **0 357 967 B1**

(12) **EUROPÄISCHE PATENTSCHRIFT**

(45) Veröffentlichungstag der Patentschrift: **02.12.92**

(21) Anmeldenummer: **89114415.6**

(22) Anmeldetag: **04.08.89**

(51) Int. Cl.5: **A61K 31/575**, A61K 31/705, A61K 47/10

(54) Phyto- und Zoosterole und deren Derivate mit verbesserter Wasserlöslichkeit.

(30) Priorität: **01.09.88 DE 3829643**

(43) Veröffentlichungstag der Anmeldung:
**14.03.90 Patentblatt 90/11**

(45) Bekanntmachung des Hinweises auf die
Patenterteilung:
**02.12.92 Patentblatt 92/49**

(84) Benannte Vertragsstaaten:
**AT BE CH ES FR GB IT LI LU NL SE**

(56) Entgegenhaltungen:
**DE-A- 2 303 247
DE-A- 2 759 171
DE-A- 3 042 975
DE-B- 1 131 845**

**Drug Dev. Ind. Pharm. 10 (1984), 771-787**

**Journal of Pharmaceutical Sciences, Band 60, Nr. 10, Oktober 1971, Seiten 1569-1571, American Pharmaceutical Association, Washington, US; W.L. Chiou et al.**

(73) Patentinhaber: **Roecar Holdings (Netherlands Antilles) N.V.
Kerkstraat 10a
Willemstad, Curacao Netherlands Antilles(NL)**

(72) Erfinder: **Koch, Heinrich P. Prof. Dr.
Kahlenberger Str. 37
A-1190 Wien(AT)**

(74) Vertreter: **Siewers, Gescha, Dr.
Rechtsanwälte Dr. Harmsen, Dr. Utescher Dipl.-Chem. Harmsen, Bartholatus Dr. Schaeffer, Dr. Fricke, Wolter Patentanwalt Dr. Siewers Adenauerallee 28
W-2000 Hamburg 1(DE)**

EP 0 357 967 B1

**Beschreibung**

Phytosterole und ihre Derivate, und zwar Glykoside und Ester, kommen in der Natur in fast allen Pflanzen und häufig auch in Mikroorganismen vor. Das bekannteste Zoosterol ist Cholesterol, das zusammen mit seinen Derivaten in pflanzlichem und tierischem Gewebe vorkommt. Im folgenden werden unter Phytosterole auch Cholesterol und seine Derivate verstanden, da sie im pharmazeutischen-technologischen Sinne mit den Phytosterolen voll vergleichbar sind.

Zu den in der Natur am häufigsten vorkommenden Phytosterolen gehört Sitosterol, das in der Regel aus dem unverseifbaren Teil verschiedener pflanzlicher Öle gewonnen wird. Synthetisch können Glykoside des Sitosterol und der übrigen Phytosterole auch nach der bekannten Königs-Knorr-Synthese unter Verwendung der entsprechenden Aglycone und eines am $C_1$ bromierten Zuckerazetates unter Verwendung von Silber- oder Kadmiumkatalysatoren in relativ großer Ausbeute hergestellt werden. Das natürlich oder halbsynthetisch gewonnene Sitosterol enthält nicht nur ß-Sitosterol, sondern in der Regel auch mehr oder weniger kleine Mengen chemisch nahe verwandter Sterole, insbesondere Stigmasterol und Campesterol. In der Natur werden die Sterole in der Regel durch einen kleinen Anteil ihrer Glykoside oder Ester begleitet, die generell als Steroline bezeichnet werden. Die Steroline sind meist Monoglykoside, obgleich auch einige Oligoglykoside bekannt geworden sind. In natürlichem Material liegen die Steroline und auch die Stercle zum Teil auch in Form der Ester, und zwar überwiegend mit Monocarbonsäuren vor.

Sowohl Sitosterol als auch Sitosterolin werden therapeutisch eingesetzt, und zwar als antilipaemische oder anticholesterolaemische Mittel. Außerdem werden die Verbindungen zur Behandlung der benignen Prostatahyperplasie eingesetzt.

Die Phytosterole und ihre Derivate sind in der Regel relativ schwer wasserlöslich; dies trifft auch auf das Sitosterol bzw. das Sitosterolin zu. Daher werden Sitosterol bzw. sein Glukosid bisher in der Regel nur oral in Form von Gelatinekapseln oder Tabletten verwendet. Die Bioverfügbarkeit der Drogen ist aber bei diesen klassischen Darreichungsformen nur recht gering aufgrund der mangelnden Wasserlöslichkeit und kann daher nur durch eine relativ hohe Dosierung der aktiven Wirkstoffe ausgeglichen werden.

Zu den geläufigsten Verfahren, um schwer wasserlösliche Wirkstoffe besser verfügbar zu machen, gehört die Mikronisierung, bei der Kristallpartikel in der Größenordnung von Mikrometer anfallen. Bei den hier in Frage stehenden Wirkstoffen hat sich das Verfahren aber nicht bewährt, da trotzdem die Resorptivität relativ gering ist. Andere bekannte Verfahren zur Verbesserung der Wasserlöslichkeit lipophiler Wirkstoffe sind das Mischschmelzverfahren, bei denen die gemischte Schmelze aus Wirkstoff und Träger bei schneller Abkühlung der Schmelze eine homogene Mischung oder eine feste Lösung bildet. Eine ähnliche Technologie liegt bei der Herstellung sogenannter Kopräzipitate vor, bei der Wirkstoff und Träger in einem geeigneten Lösungsmittel gelöst und das Lösungsmittel anschließend zur Trockne abgedampft wird, wobei sich ein Produkt bildet, das ähnliche Eigenschaften wie eine feste Lösung aufweist und als Kopräzipitat bezeichnet wird. In beiden Fällen enthalten die Feststoffe den Wirkstoff in molekularer Verteilung im Träger. Wenn der Träger selbst wasserlöslich ist, wird bei der Auflösung in Wasser der Wirkstoff in der feinsten Partikelform freigesetzt, die überhaupt nur erreicht werden kann. Auf diese Art und Weise erhöht sich die Lösungsgeschwindigkeit des Arzneimittels in Wasser um mehrere Größenordnungen und damit steigt in der Regel auch die Bioverfügbarkeit.

Als Träger oder feste Lösungsmittel für lipophile Arzneistoffe sind schon eine Reihe von Verbindungsklassen vorgeschlagen worden, so unter anderem Zucker, Zuckeralkohole, organische Säuren, Harnstoffe, Polyanhydride, Polyamide, Polyacrylate und Polyacrylamide, Polyester, PVP und auch Polyethylenglykole unterschiedlichem Molekulargewichts. Es hat sich aber herausgestellt, daß es kein Schema gibt, wonach die Verwendbarkeit einer bestimmten Substanzgruppe als Träger für einen bestimmten Wirkstoff voraussagbar ist, selbst wenn man von vornherein beachtet, daß Träger und Wirkstoff bei der Herstellung von festen Lösungen Schmelzpunkte im etwa vergleichbaren Bereich haben müssen, da sonst bei Überhitzung eines Bestandteiles bereits Zersetzungserscheinungen auftreten können. Vielmehr hat sich gezeigt, daß völlig unerwartete Inkompatibilitäten bei der Herstellung auftreten können und daß zum Teil die Stabilität des Wirkstoffes selbst durch eine Einbettung negativ beeinflußt werden kann ; so ist beispielsweise bekannt, daß Polyethylenglykole und verwandte Verbindungen zur Peroxydbildung neigen und damit auch die oxidative Stabilität von Wirkstoffen verringern können. Außerdem hat sich herausgestellt, daß in vielen Fällen die Lagerstabilität solcher Einbettungen oder Kopräzipitate nicht in hinreichendem Maße gegeben ist, da Rekristalisation und damit Vergrößerung der Partikel, Übergang in andere Kristallformen usw. eintreten können. Voraussetzung für eine pharmazeutische Verwendbarkeit ist aber, daß neben einer erhöhten Wasserlöslichkeit und damit besseren Bioverfügbarkeit auch eine hinreichend lange Stabilität und somit Lagerfähigkeit der Produkte erzielt wird.

Polyethylenglykole, im folgenden als PEG abgekürzt, sind schon häufiger zur Verbesserung der Verfügbarkeit von schwerlöslichen Arzneistoffen herangezogen worden. Während die Schmelzeinbettung oder Kopräzipitation mit PEG bei einer Reihe von Arzneimitteln die Resorptivität und die Bioverfügbarkeit verbessern, ist beispielsweise auch bekannt, daß bei Barbituraten die Löslichkeit und auch die Resorption durch die Verarbeitung mit PEG deutlich reduziert werden; das gleiche gilt für Chlorthalidon, Salicylsäure, Griseofulvin, Disulfiram, Frusemid, Chlorothiazid und andere Arzneistoffe.

Auch bei der Verarbeitung von Steroid - Verbindungen sind völlig unterschiedliche Ergebnisse erzielt worden; während beispielsweise Hoelgaard et al. in Arch.Pharm.Chemie Sci.Ed.3 1975, 34 - 47 angeben, daß durch PEG-Einbettung die Auflösungsgeschwindigkeit von Testosteron sehr stark vergrößert werden kann, wenngleich auch beim Lagern Partikelvergrößerungen zu beobachten sind, und Chiou et al. in J. Pharm. Sci. 60, 10, 1569 (1971) angeben, daß die Auflösungsgeschwindigkeit von Digitoxin, Methyltestosteron, Hydrocortison und weiteren Corticosteroiden bei Dispersion in Polyethylenglykol 6000 verbessert wird, steht dem auf der anderen Seite die Feststellung gegenüber, daß durch PEG-Verarbeitung Prednison, Prednisolon und auch Hydrocortison eine mangelhafte Stabilität aufweisen, wie von Khalil et al. in Drug Dev. Ind. Pharm. 10 (1984) 771,787 festgestellt. Aus dieser Veröffentlichung ergibt sich, daß die Verwendung von PEG für Einbettungspräparate für Prednisolon, Prednison und Hydrocortison keineswegs unproblematisch ist. Auch Chiou gibt an, daß Digitoxin nach Einbettung in PEG teilweise zersetzt wird.

Völlig überraschend wurde jetzt festgestellt, daß sich Phytosterole und deren Derivate wie Glykoside oder Glykosidester besser wasserlöslich machen lassen und eine hervorragende Stabilität aufweisen, wenn sie als feste Lösungen oder Kopräzipitate mit Polyethylenglykolen mit einem Molekulargewicht zwischen etwa 200- 50.000 vorliegen, und zwar vorzugsweise als 1-4%ige feste Lösung.

Nach den bisherigen Erfahrungen war nicht zu erwarten, daß sich Phytosterole im Hinblick auf die schon bekannten Schwierigkeiten mit Steroiden wie Prednison, Prednisolon oder Hydrocortison unbedenklich mit PEG verarbeiten lassen würden, da auch diese Substanzen relativ empfindlich sind. Außerdem weisen sie zumeist Polymorphismus auf, so daß zumindest beim Lagern mit Umwandlungen und Kornvergrößerungen zu rechnen gewesen wäre.

Es hat sich aber herausgestellt, daß die Verarbeitung von Phytosterolen und ihren Derivaten sowohl durch Schmelzeinbettung wie durch Herstellung von Kopräzipitaten möglich ist. Die dabei entstehenden Sterol-PEG-Mischungen zeigen eine um Größenordnungen höhere Auflösungsgeschwindigkeit als die Ursprungssubstanzen und außerdem eine für technologische Zwecke hinreichende Lagerfähigkeit. Zersetzungen oder oxidative Veränderungen der Substanzen wurden nicht beobachtet.

Die Herstellung erfolgt in an und für sich bekannter Weise, indem PEGs mit unterschiedlichem Molekulargewicht und die Sterole oder ihre Derivate fein verrieben und gemischt und auf eine Temperatur erwärmt werden, die oberhalb der Schmelztemperatur der Komponenten liegt, wobei sich eine klare glasartige Schmelze formt. Diese Schmelze wird dann abrupt durch Ausgießen auf gekühlte Stahl- oder Glasoberflächen abgekühlt und die sich dabei bildende feste Masse vermahlen und in üblicher Weise zu pharmazeutischen Zubereitungen weiterverarbeitet. Man kann aber auch das PEG und das Sterol oder ein Derivat in einer möglichst kleinen Menge eines niederen Alkohols wie beispielsweise Methanol, Ethanol, Isopropanol oder ähnlichem auflösen, bis sich eine klare Lösung gebildet hat. Diese Lösung wird dann im Vakuum abgedampft, und zwar solange, bis der Rückstand von jedem Lösungsmittel befreit ist. Die verbleibende feste Masse wird dann wie bei der Schmelzeinbettung weiterverarbeitet.

Die erfindungsgemäß hergestellten Zubereitungen können für orale Darreichungsformen eingesetzt werden wie beispielsweise Tabletten, film- oder zuckerbeschichtete Tabletten, Hart- oder Weichgelatinekapseln oder ähnliches, oder sie können auch für rektale oder perkutane Applikationsformen Anwendung finden. Zu den geeigneten rektalen Anwendungsformen gehören Suppositorien, Globuli, Rektalcremes oder Rektalkapseln. Gegebenenfalls kann man die vorbereiteten Verbindungen auch in Salbenform zur Anwendung bringen, indem sie in üblicher Weise in Salben oder Cremes eingearbeitet werden.

Die Erfindung wird im folgenden anhand der Beispiele näher erläutert:

Beispiel 1

3,9g PEG MG 4.000 und 0,1g Sitosterol oder Sitosterolin werden fein verrieben, gemischt und bis zu einer Temperatur oberhalb der Schmelztemperaturen der Komponenten erwärmt, bis sich eine klare glasartige Schmelze bildet. Die Schmelze wird dann durch Ausgießen auf eine ausgekühlte Stahl- oder Glasfläche schockartig abgekühlt. Die verfestigte Masse wird dann zu einem Pulver mit einer Korngröße von 0,149-0.074 mm (100 - 200) mesh vermahlen. Anschließend wird das Pulver in Hartgelatinekapseln in einer Menge von jeweils 400 mg eingefüllt.

Auflösungsversuche wurden anschließend nach dem bekannten Verfahren mit rotierender Scheibe oder mit Rührer durchgeführt.50% der eingesetzen Menge der Mischung lösten sich zwischen 3 - 22 Minuten, je nach dem welche Art des PEG eingesetzt wird. Lösungsmittel war Wasser mit einer Temperatur von 37 °C. Völlige Auflösung wurde bei allen Versuchen innerhalb von 30 Minuten erzielt, wobei sich in jeden Fall auch eine klare Lösung bildete.

Beispiel 2

3,9 g Polyethylenglykol MG 10.000 und 0,1 g Sitosterol oder Sitosterolin wurden in einer ausreichenden Menge Methanol oder Ethanol aufgelöst, bis sich eine klare Lösung bildete. Anschließend wurde das Lösungsmittel im Vakuumverdampfer abgezogen. Der Rückstand wurde solange abgedampft, bis er vollständig lösungsmittelfrei war. Die verbleibende feste Masse wurde dann weiter wie in Beispiel 1 verarbeitet.

Bei den Auflösungsversuchen zeigten sich keinerlei Unterschiede zu den Einbettungsverbindungen.

Entsprechend den Beispielen 1 und 2 wurden weitere Versuche mit PEG mit Molekulargewichten von 2000, 6000 und 20.000 durchgeführt, die ebenfalls vergleichbare Ergebnisse erbrachten. Für perorale Arzneimittelform wird vorzugsweise ein PEG mit einem Molekulargewicht von 4000 und für Suppositorien ein solches mit einem Molekulargewicht von 6000 eingesetzt, da hier eine besonders gute Verarbeitbarkeit gegeben ist.

**Patentansprüche**

1. Arzneimittel mit einem Gehalt an Phytosterolen oder deren Derivaten mit verbesserter Wasserlöslichkeit, dadurch gekennzeichnet, daß die Phytosterole oder deren Derivate als feste Lösung oder Kopräzipitate jeweils mit Polyethylenglykolen mit Molekulargewichten zwischen 200 bis 50.000 vorliegen.

2. Arzneimittel nach Anspruch 1, dadurch gekennzeichnet, daß die Wirkstoffe als 1-4%ige feste Lösung oder Kopräzipitat vorliegen.

3. Arzneimittel nach Anspruch 1, dadurch gekennzeichnet, daß die Wirkstoffe in Form ihrer Glykoside, Ester oder Glykosidester vorliegen.

4. Arzneimttel nach Anspruch 1, dadurch gekennzeichnet, daß die Wirkstoffe als Glykoside vorliegen.

5. Arzneimittel nach Anspruch 1 bis 4 zur oralen oder rektalen Behandlung von Lipidaemie, Cholesterolaemie oder benigner Prostatahyperplasie.

**Claims**

1. Drugs with a content of phytosterols or their derivatives with improved solubility, characterised in that the phytosterols or their derivatives are present as solid solution or coprecipitates, in each case with polyethylene glycols having molecular weights between 200 and 50.000.

2. Drugs according to Claim 1, characterised in that the effective substances are present as 1-4% solid solution or coprecipitates.

3. Drugs according to Claim 1, characterised in that the effective substances are present in the form of their glycosides, esters or glycoside esters.

4. Drugs according to Claim 1, characterised in that the effective substances are present as glycosides.

5. Drugs according to Claims 1 to 4 for the oral or rectal treatment of lipidaemia, cholesterolaemia or benign prostata hyperplasia.

**Revendications**

1. Médicaments contenant des phytostérols ou ses dérivés d'une solubilité améliorée caractérisés par le fait que les phytostérols ou ses dérivés se trouvent sous forme de solution solide ou coprécipitatés chacun comprenant des glycols polyéthylènes d'un poids moléculaire situé entre 200 et 50.000.

2. Médicaments selon revendication 1 caractérisés par le fait que les agents actifs se trouvent sous forme de solution solide 1-4% ou coprécipitatés.

3. Médicaments selon revendication 1 caractérisés par le fait que les agents actifs se trouvent sous forme de leurs glycosides, esters ou glycosidesters.

4. Médicaments selon revendication 1 caractérisés par le fait que les agents actifs sont présents en tant que glycosides.

5. Médicaments selon revendications 1 à 4 pour le traitement oral et rectal de la lipidémie, la cholestérolémie ou de l'hyperplasie bégnine de la protaste.